# EUROPEAN PATENT SPECIFICATION

(11) **EP 4 192 554 B1**
(45) Date of publication and mention of the grant of the patent: **12.03.2025**
(21) Application number: 21752241.6
(22) Date of filing: 03.08.2021
(51) Int. Cl.: A61M 11/02, A61M 15/00, A61M 15/02, A61M 16/14, A61M 16/20

(54) **DELIVERY DEVICE FOR PRODUCT TO BE INHALED**
ABGABEVORRICHTUNG FÜR INHALIERBARE PRODUKTE
DISPOSITIF D'ADMINISTRATION POUR PRODUITS À INHALER

(30) Priority: 10.08.2020 IT 202000019873
(43) Date of publication of application: 14.06.2023
(73) Proprietor: Giacalone, Tommaso, Viale Papa Giovanni XXIII Carpenedolo (BS) (IT); Hungar, Janos Mikael, 25015 Desenzano del Garda (BS) (IT)
(72) Inventor: Giacalone, Tommaso, Viale Papa Giovanni XXIII Carpenedolo (BS) (IT); Hungar, Janos Mikael, 25015 Desenzano del Garda (BS) (IT)
(74) Representative: Busca, Andrea
(86) International application number: PCT/IB2021/057076
(87) International publication number: WO 2022/034431

(56) References cited:
- EP-A1- 2 457 559
- EP-A1- 2 767 301
- EP-A2- 0 166 476
- WO-A1-2010/122103
- WO-A1-2016/001926
- WO-A1-2016/117831
- WO-A2-2015/127147
- RO-B1- 122 128
- US-A1- 2009 232 895
- US-A1- 2019 083 395
- US-A1- 2019 321 570
- US-B2- 8 662 078

## Description

The present invention relates to a unit for delivering a product to be inhaled. The present invention also relates to a delivery unit.

In the treatment of certain diseases affecting the respiratory tract, it is known to administer a substance, such as a drug containing the active ingredient, in the form of an aerosol. For this type of administration, there are devices that make it possible to nebulise the drug after mixing it with a physiological solution. Such equipment includes a mouthpiece or mask for delivering the nebulised drug. It is also known to administer drugs via inhalers, in which the drug is not nebulised but aspirated directly by the user.

More recently, natural treatment methods have been developed involving the use of sodium chloride ("halotherapy"), which is "micronised", i.e. finely pulverised into particles having a size such as between 4 and 40 micrometres in size.

Halotherapy basically consists in inhaling an aerosol consisting of micronised sodium chloride. In a halotherapy session, the user is in a "salt chamber" where the atmosphere is saturated with sodium chloride, using salt micronising devices that dry-reduce pharmaceutical grade salt into micrometre-sized particles, which are then ionised. The micronising device is usually programmed to synchronise particle size, concentration, temperature (18°-24) and humidity (40-60%) by mixing the micronised salt with a regulated stream of air, which is then evenly dispersed throughout the salt chamber. The tiny negatively ionised salt particles create an aerosol, which is able to travel deep into the respiratory system and even reach the lungs at alveolar level.

The known devices have several drawbacks.

Delivering medical salt by aerosol does not allow to control the actual salt uptake by the user.

In addition, the treatments involve the use of a mixture of salts having different grain sizes. Therefore, it is difficult to determine the optimal dosage of the salts in the mixture, which varies depending on the user.

Recently, "helmets" for the assisted ventilation, e.g. in the continuous positive airway pressure (CPAP) ventilation, have often been used to treat breathing difficulties. However, it is difficult to use a product to be associated with such ventilation. Furthermore, it is not possible to control the dosage of such product.

US2019/321570, US2009/232895, EP2767301 are known from the Patent Literature. However, they are suitable for therapies that are extremely different from halotherapy, as they require interventions on the product to make it inhalable, such as vaporisation, heating, etc. Thus, they represent a diversion towards devices which do not require any product treatment.

The object of the present invention is to overcome all or some of the drawbacks of the prior art.

A preferred object of the present invention is to enable a product used in a treatment to be better assimilated.

A further object of the present invention is to allow an optimal dosage of the product depending on the user.

According to the present invention, a delivery unit having the characteristics defined in claim 1 is provided.

The delivery unit allows the product to be picked up by the user aspiring the product, and to control the delivery depending on the aspiration force. The product is therefore completely assimilated by the user, without any dispersion.

According to an unclaimed aspect, an inhalation cartridge is provided.

The filter at the outlet opening is a selective filter to prevent crystals larger than a predetermined threshold from exiting and to allow air to exit, while the filtering material placed to close the perforation preferably only allows the passage of air but not of the crystals contained in the cartridge.

The cartridge contains the mixture suitable for a particular treatment, and does not need to be calibrated each time.

According to another unclaimed aspect , an inhalation kit is provided, comprising the delivery device and the cartridge containing the suitable mixture, which is connected to the delivery device to be optimally administered.

The delivery unit can be associated with an assisted ventilation system, particularly of the "CPAP" type, allowing treatment to be carried out even during ventilation by controlling the dosage of the product.

Further advantages and features of the present technology will be more apparent in the following detailed description, made with reference to the accompanying drawings, which represent a non-limiting example thereof, wherein:
- Figure 1 shows a delivery device according to the present technology in a first preferred embodiment in a first configuration;
- Figure 2 shows the delivery device of Figure 1 in a second configuration;
- Figure 3 shows a compensation valve of the delivery device in a second embodiment in a section view;
- Figure 4 shows a guide support of the valve of Figure 3 in a top view;
- Figure 5 shows a variant of the compensation valve of Figure 3 in a section view;
- Figure 6 shows a guide support of the valve of Figure 5 in a perspective view;
- Figure 7 shows a variant of the compensation valve of Figure 5 in a section view;
- Figure 8 shows a guide support of the valve of Figure 7 in a perspective view;
- Figure 9 shows a cartridge for a product to be inhaled in a preferred embodiment;
- Figure 10 schematically shows an assisted ventilation system in which the delivery device is inserted;
- Figure 11 shows a cartridge for a product to be inhaled that can be used in a delivery unit.

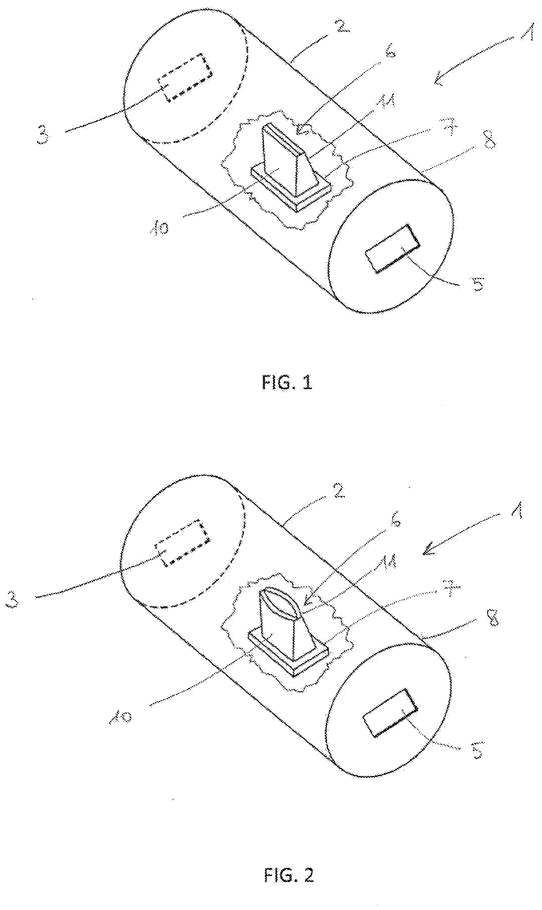

In Figure 1, a device for delivering at least a product to be inhaled is referred to as 1. The device 1 is used in particular for inhaling micronised salt crystals.

The delivery device 1 comprises at least one chamber 2 having a first inlet opening 3 adapted to be connected to a cartridge 4 containing at least a product to be inhaled and a second outlet opening 5 adapted to be connected to an element for aspiring the product. The second opening 5 is arranged at a predetermined distance from the first opening 3.

According to the technology, the delivery device 1 comprises at least one compensation valve 6 arranged at a third opening 7 made on a wall 8 of the chamber 2, wherein the compensation valve 6 is adapted to maintain a closed configuration when the pressure inside the chamber 2 is substantially equal to the pressure outside the chamber 2 at the first inlet opening 3 and an open configuration when the pressure inside the chamber 2 is below a predetermined value.

The compensation valve 6 is shaped so as to have an opening proportional to the difference of pressure existing inside the chamber 2 and outside the chamber 2 at the inlet opening 3.

The pressure inside the chamber is therefore maintained at a constant value during aspiration. This allows a constant dose of product to be delivered regardless of the inhalation force exerted by the user.

When the pressure inside chamber 2 is equal to the external pressure, i.e. when inhalation by the user does not occur, the valve 6 is closed. When inhalation occurs, the valve 6 opens and the opening increases proportionally with respect to the aspiration force, i.e. the difference of pressure generated inside the chamber 2.

In a first embodiment, the delivery device 1 is used as a common inhaler, in which aspiration occurs via a mouthpiece.

In this embodiment, the compensation valve 6 connects the inside of chamber 2 directly with the outside environment. When inhalation occurs, the valve 6 opens and the opening increases proportionally to the negative pressure generated inside chamber 2.

In other words, if the aspiration force is low, for instance because
exerted by a child, the valve tends to stay closed, so that the entire dose is inhaled. If, on the other hand, the aspiration force is high, the valve opens wide enough to generate a significant flow of air into the chamber, to prevent the delivery of an excessive dose of product.

Figure 1 shows a first type of compensation valve of the duckbill check valve type. The valve 6 comprises a first lip 10 and a second lip 11 adjacent to each other in a closed configuration. The valve 6 is advantageously made of an elastic material, such as rubber.

In a closed configuration (figure 1), the valve 6 has a substantially trapezoidal cross-section, with the larger base open arranged at the third opening 7 obtained on the wall 8 of the chamber 2. The shorter base consists of the ends of the first lip 10 and the second lip 11 in an adjacent position, and is arranged inside the chamber 2.

In the illustrated embodiment, when inhalation does not occur, the first lip 10 is in contact with the second lip 11, and the valve 6 is closed.

When inhalation occurs, depending on the aspiration force by the user, the pressure inside the chamber 2 decreases, and the valve 6 opens allowing air to enter the chamber (figure 2). The valve 6 is calibrated in such a way that the greater the inhalation, the greater the opening of the valve 6, so that the negative pressure inside the chamber 2 during inhalation is kept at a constant predefined value and the same dose is always delivered.

The predefined value is determined on the basis of various parameters such as the size of the valve, the thickness of the first and second lip and the flexibility of the valve material.

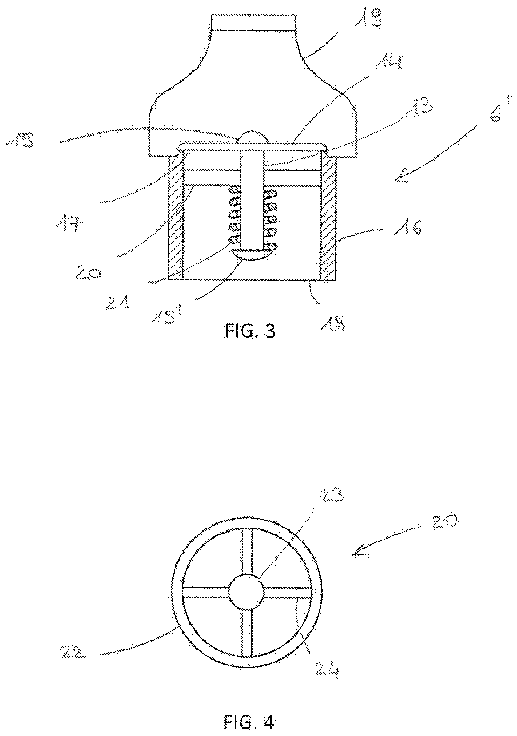

Figure 3 shows a second type of compensation valve, of the poppet valve type.

The poppet valve 6' has a stem 13 having a substantially flat head 14 arranged at a first end 15 of the stem 13.

The valve 6' has a containment 16, preferably circular, having a first open end 17 connected to the chamber 2 of the delivery device 1 at the third opening 7 and a second open end 18. The head 14 of the valve 6' is arranged at the opening of the first end 17 of the containment 16. In the embodiment of figure 3, the first end 17 of the containment 16 is advantageously associated with a connecting pipe 19 with the third opening 7 of the chamber 2.

A guide support 20 of the stem 13 is arranged within the containment 16. The return action is advantageously exerted by a spring 21, such as a coil spring, interposed between the guide support 20 and the second end 15' of the stem 13.

In the embodiment shown, the valve is arranged outside the chamber. In an alternative embodiment, not shown, the valve is arranged inside the chamber.

In the embodiment of figure 3, when inhalation does not occur, the head 14 of the valve 6' is held by the spring 21 resting on the first end 17 of the containment 16, closing the passage to the third opening 7 of the chamber 2 of the delivery device 1.

When inhalation occurs, a force is exerted which brings the head 14 of the valve 6' away from the first end 17 of the containment 16 opening the passage to the inside of the chamber 2. The passage of air from outside the chamber 2 is proportional to the force exerted. Some parameters such as the opening size, the material of the head, the spring and the stem length are selected to maintain a constant negative pressure within the chamber.

In the embodiment shown, the containment 16 of the valve 6' is substantially cylindrical. The opening of the first end 17 of the containment 16 and the head 14 of the valve are substantially circular.

In the embodiment shown in Figure 4, the guide support 20 is disc-shaped, preferably having an outer ring 22 and an inner ring 23 and connecting arms 24, for example four, between the outer ring 24 and the inner ring 23.

In a preferred embodiment, the compensation valve is adjustable.

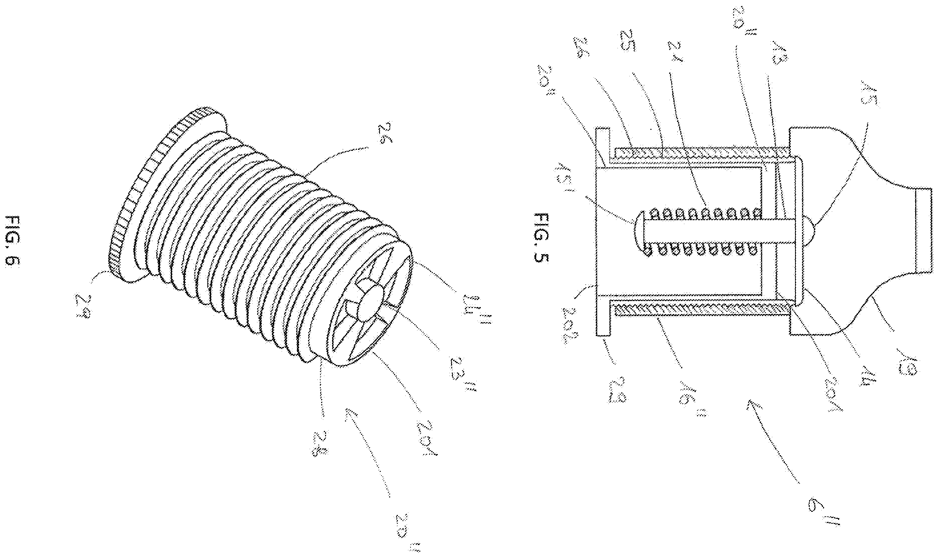

Figure 5 shows a variant 6" of the valve of figure 3, wherein the containment 16'' has an inner threaded wall 25 and the guide support 20" has a counter threaded outer wall 26, so that the guide support 20" can be screwed inside the containment 16''.

When the guide support 20'' is fully screwed in, the force of the spring 21 is minimal, whereas by unscrewing the guide support 20'' the spring 21 is compressed, thus increasing the force required to open the valve. Thereby, the preload on the spring can be varied depending on the conditions of use.

The guide support 20'' is preferably provided with a scale to indicate the set opening pressure in order to facilitate adjusting the valve based on the user.

Figure 6 shows the guide support 20'' of the valve of figure 5. The guide support 20" is substantially cylindrical and has an outer threaded wall 26. The support 29" has at one first end 201 a central ring 23'' inside which the stem 13 of the valve 6" can slide. The central ring 23'' is connected by means of four arms 24'' to the side wall 28. The second end 202 of the guide support 20" is open and surrounded by a flange 29.

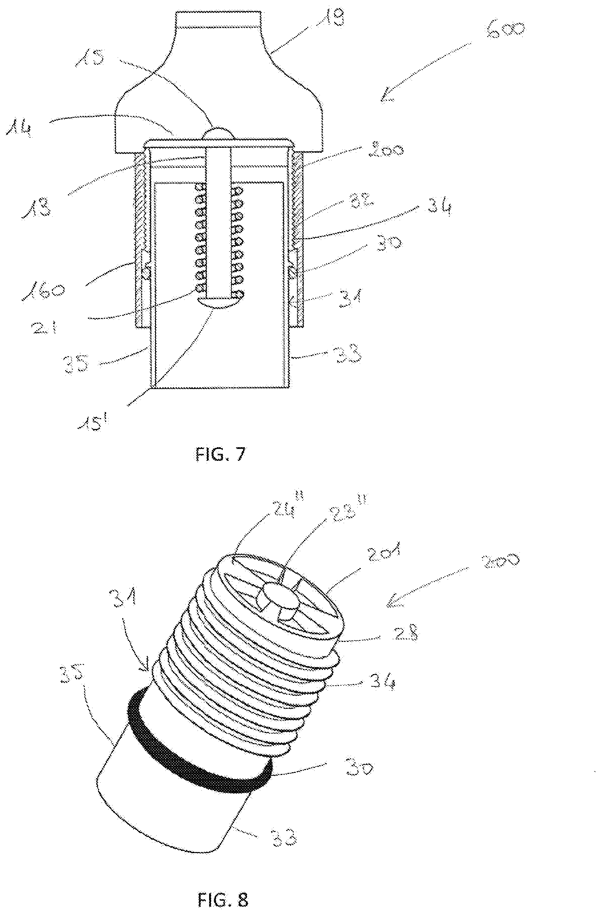

Advantageously, the poppet valve 6', 6'' has a sealing ring 30, such as an O-ring, arranged between the outer side wall 31 of the guide support 20, 20" and the inner wall 32 of the containment 16, 16'' of the valve 6', 6'' (figure 7).

In an advantageous embodiment, the guide support 20, 20'' has an extension 33 extending beyond the containment 16, 16'' of the valve 6', 6'' (figure 8).

Figure 7 shows a variant of the valve of figure 5, wherein the valve 6'' has a sealing ring 30, such as an O-ring, arranged between the outer side wall 31 of the guide support 200 and the inner wall 32 of the valve containment 160. In such an embodiment, the guide support 200 has an extension 33 extending beyond the valve containment 160.

In particular, as illustrated in figure 8, the outer wall 31 of the guide support 200 has a first threaded portion 34 and a thread-free portion 35 on which the sealing ring 21 is arranged. The thread-free portion 35, which extends outside the containment 160, allows the valve to be connected to an external pipe.

The embodiments shown in Figures 3-8 are advantageous when using the delivery device in a continuous positive airway pressure "CPAP" assisted ventilation system, as will be described below.

According to an aspect of the present disclosure, a cartridge 4 connectable to a delivery device 1 is provided.

The cartridge 4 contains at least a product to be inhaled, namely micronised salt crystals. In a preferred implementation the cartridge contains 10-20 grams of micronised salt. The size of the salt particles varies depending on the treatment to be carried out. They may be basically of four types.

The first type of 'A' particles is 0.2 to 1 mm in size and has the main function of transporting the salt particles, preventing them from aggregating.

A second type of 'B' particles is up to 5 microns in size.

A third type of 'C' particles is 5 to 10 microns in size.

A fourth type of 'D' particles is 10 to 30 microns in size.

For exemplary purposes, the cartridge 4 may contain a mixture consisting of 60% of "A" particles, 20% of "B" particles, 10% of "C" particles and 10% of "D" particles. The composition varies depending on the treatment required.

The cartridge 4 can be replaced and connected to the delivery device 1 as required.

The cartridge 4 comprises a containment element 36 with a product outlet opening 37. The cartridge 4 is advantageously made of plastic. The walls 38 of the cartridge 4 are at least partially perforated and preferably lined with a filtering material.

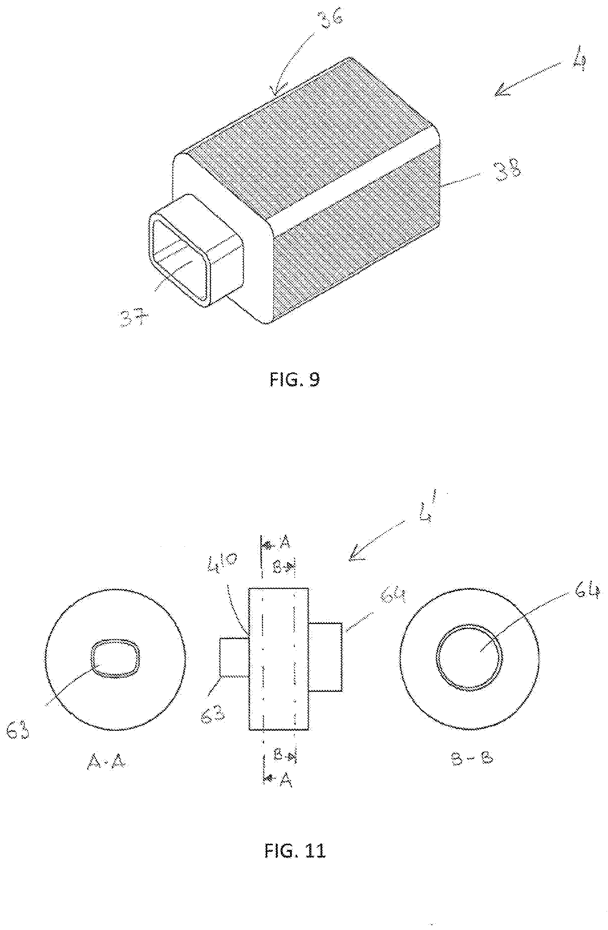

In the embodiment shown in Figure 9, the cartridge 4 has a substantially parallelepiped shape. The cartridge 4 is perforated on one or more sides to allow air to pass through. The perforated sides are advantageously lined with a filtering material to prevent salt particles from exiting.

A filter is arranged at the outlet opening 37,
preferably a 100-200 micron filter, in order to prevent larger salt particles from reaching the user's mouth.

In an embodiment not shown, the containment 36 has a door for inserting an envelope already provided with a filtering net.

According to an aspect of the present disclosure, an inhalation kit is provided comprising a delivery device 1 and a cartridge 4 to be connected to the delivery device.

According to the present invention, a delivery unit 39 of at least one product to be inhaled is provided.

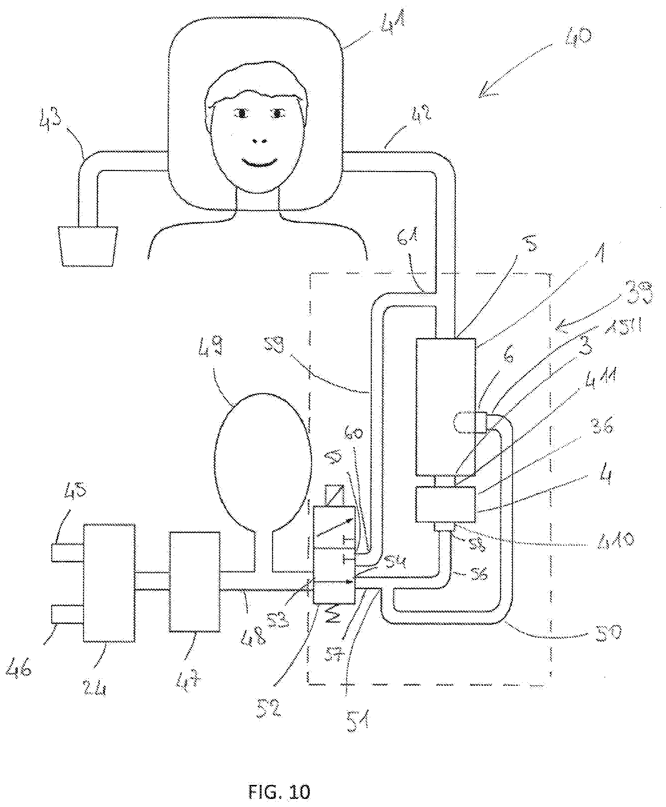

Figure 10 shows an embodiment wherein the delivery unit 39 is arranged in an assisted ventilation system 40, such as of the continuous positive airway pressure (CPAP) type. Such system 40 involves the use of a helmet 41, which allows to provide artificial ventilation to a patient with breathing difficulties. The helmet 41 is normally connected to at least two pipes:
a pipe 42 for air/oxygen supply and a discharge pipe 43.

The system 40 comprises a gas mixer 44 with an oxygen inlet duct 45 and an air inlet duct 46. The gas leaving the mixer 44 is at a pressure of about 20 mbar and is led to a humidifier/heater 47. The outlet pipe 48 from the humidifier/heater is connected to a compensation tank 49 to maintain a constant pressure.

The pipe 48 is connected to the delivery unit 39.

According to the invention, the delivery unit 39 comprises a delivery device 1, wherein the second outlet opening 5 is adapted to be connected to a first pipe 42; at least one cartridge 4 comprising a containment element 36 for at least a product to be inhaled, in particular comprising micronised salt crystal particles of different sizes, having an inlet 410 adapted to be connected to a second pipe 48 and an outlet 411 associated with the inlet opening 3 of the delivery device 1; and a first compensation duct 50 having a first end 51 adapted to connect the second pipe 48 upstream of the cartridge 4 and a second end 511 connected to the compensation valve 6 of the delivery device 1.

In the embodiment shown in Figure 10, the delivery unit 39 advantageously comprises a by-pass valve 52 having an inlet 53, a first outlet 54 and a second outlet 55, wherein the inlet 53 is adapted to be connected to the second pipe 48; a second supply duct 56 having a first end 57 connected to the first outlet 54 of the by-pass valve 52 and a second end 58 connected to the inlet 410 of the cartridge **4;** and a third by-pass duct 59 having a first end 60 connected to the second outlet 55 of the by-pass valve 52 and a second end 61 adapted to be connected to the first pipe 42.

In particular, the by-pass valve 52 has an inlet 53 adapted to be connected to the pipe coming from the humidifier/heater 48. When the by-pass valve 52 is closed, the air/oxygen mixture is sent directly to the pipe 42 connected to the helmet. When the by-pass valve 52 is open, the pressurised air/oxygen mixture passes through the cartridge 4 picking up the product and enters the chamber 2 of the delivery device 1.

Upon each inhalation by the user, the pressure inside the chamber 2 of the delivery device 1 decreases and the pressure change causes the compensation valve 6 to open. The valve 6 is sized to maintain a constant difference of pressure within chamber 2 during inhalation, so that the correct dose of product can be inhaled.

It is possible to administer the product to the user only when require by adjusting the opening and closing of the by-pass valve 52.

Figure 11 shows an embodiment of the cartridge 4', which can be used in particular in the delivery unit.

The cartridge 4' has a first substantially rectangular-shaped adapter 63 placed at the inlet 410, to be connected to the supply duct 56. The cartridge 4' further has a second substantially circular-shaped output adapter 64, to be connected to the first inlet opening 3 of the delivery device 1.

Advantageously, in the delivery unit 39 the delivery device 2 described with reference to Figures 7-8 may be used, wherein the extension 33 of the guide support 200 enables the connection with the compensation duct 50.

### GENERAL INTERPRETATION OF TERMS

In understanding the object of the present invention, the term "comprising" and its derivatives, as used herein, are intended as open-ended terms that specify the presence of declared characteristics, elements, components, groups, integers and/or steps, but do not exclude the presence of other undeclared characteristics, elements, components, groups, integers and/or steps. The above also applies to words, which have similar meanings, such as the terms "comprised", "have" and their derivatives. Furthermore, the terms "part", "section", "portion", "member" or "element" when used in the singular can have the double meaning of a single part or a plurality of parts. As used herein to describe the above executive embodiment(s), the following directional terms "forward", "backward", "above", "under", "vertical", "horizontal", "below" and "transverse", as well as any other similar directional term, refers to the embodiment described in the operating position. Finally, terms of degree, such as "substantially", "about" and "approximately", as used herein, are intended as a reasonable amount of deviation of the modified term such that the final result is not significantly changed.

Various modifications and variations can be made without departing from the scope of the invention as defined in the appended claims.

## Claims

1. A unit for delivering at least one product to be inhaled comprising a delivery device,
wherein said delivery device comprises at least one chamber (2) having a first inlet opening (3) adapted to be connected to a cartridge (4) containing at least a product to be inhaled, a second outlet opening (5) adapted to be connected to at least one product aspirating element, the second outlet opening (5) being arranged at a predetermined distance from the first inlet opening (3), wherein the device further comprises at least one compensation valve (6) arranged at a third opening (7) made on a wall (8) of the chamber (2), wherein the compensation valve (6) is adapted to maintain a closed configuration when the pressure inside the chamber (2) is substantially equal to the pressure in a first compensation duct (50) and an open configuration when the pressure inside the chamber (2) is lower than a predetermined value,
wherein the second outlet opening (5) is adapted to be connected to a first pipe (42); at least one cartridge (4) comprising a containment element (36) for a product to be inhaled, in particular micronized salt crystal particles of different sizes, having an inlet (410) adapted to be connected to a second pipe (48) and an outlet (411) adapted to be associated with the inlet opening (3) of the delivery device (1); and the first compensation duct (50) having a first end (51) adapted to be connected to the second pipe (48) upstream of the cartridge (4) and a second end (51) connected to the valve (6) of the delivery device (1).

2. Unit according to claim 1, **characterized in that** the opening of the valve (6) is proportional to the difference of pressure existing inside the chamber (2) and outside the chamber (2) at the first inlet opening (3), so as to maintain the pressure inside the chamber (2) at a constant value.

3. Unit according to claim 1 or 2, **characterised in that** the opening of the valve (6) is adjustable.

4. Unit according to any one of the preceding claims 1 to 3, **characterized in that** the valve is of the poppet type (6', 6", 600), comprising a containment (16, 16", 160), preferably circular, having a first open end (17) connected to the chamber (2) of the delivery device (1) at the third opening (7) and a second open end (18), a stem (13) having a substantially flat head (14) arranged at a first end (15) of the stem (13) and arranged at the opening of the first end (17) of the containment (16, 16", 160), a guide support (20, 20", 200) for the stem (13) arranged inside the containment (16, 16", 160), and a spring (21), interposed between the guide support (20, 20'', 200) and the second end (15') of the stem (13).

5. Unit according to claim 4, **characterized in that** the containment (16", 160) of the valve has an at least partially threaded inner wall (25) and the guide support (20", 200) of the stem (13) has an at least partially counter-threaded outer wall (26, 34), so as to be screwed inside the containment to vary the spring preload.

6. Unit according to any one of the preceding claims 1 to 3, **characterized in that** the compensation valve (6) is of the duckbill check valve type and is inserted inside the chamber (2).

7. A delivery unit according to any one of the preceding claims 1 to 6, **characterized in that** it comprises at least one by-pass valve (52) having an inlet (53), a first outlet (54) and a second outlet (55), wherein the inlet (53) is adapted to be connected to the second pipe (48); a second supply duct (56) having a first end (57) connected to the first outlet (54) of the by-pass valve (52) and a second end (58) adapted to be connected to the inlet (410) of the cartridge (4); and a third by-pass duct (59) having a first end (60) connected to the second outlet (55) of the by-pass valve (52) and a second end (61) adapted to be connected to the first pipe (42).

## Patentansprüche

1. Einheit zur Abgabe von mindestens einem zu inhalierenden Produkt, die eine Abgabevorrichtung umfasst,
wobei die Abgabevorrichtung mindestens eine Kammer (2) umfasst, die eine erste Einlassöffnung (3), die mit einer Patrone (4) verbunden werden kann, die mindestens ein zu inhalierendes Produkt **enthält,** und eine zweite Auslassöffnung (5) aufweist, die mit mindestens einem Produktansaugelement verbunden werden kann, wobei die zweite Auslassöffnung (5) in einem vorbestimmten Abstand von der ersten Einlassöffnung (3) angeordnet ist, wobei die Vorrichtung ferner mindestens ein Kompensationsventil (6) umfasst, das an einer dritten Öffnung (7) angeordnet ist, die an einer Wand (8) der Kammer (2) ausgebildet ist, wobei das Kompensationsventil (6) so angepasst ist, dass es eine geschlossene Konfiguration beibehält, wenn der Druck im Inneren der Kammer (2) im Wesentlichen gleich dem Druck in einer ersten Kompensationsleitung (50) ist, sowie eine offene Konfiguration, wenn der Druck im Inneren der Kammer (2) niedriger als ein vorgegebener Wert ist,
wobei die zweite Auslassöffnung (5) mit einem ersten Rohr (42) verbunden werden kann; wobei mindestens eine Patrone (4) ein Aufnahmeelement (36) für ein zu inhalierendes Produkt, insbesondere mikronisierte Salzkristallpartikel unterschiedlicher Größe umfasst, mit einem Einlass (410), der mit einem zweiten Rohr (48) verbunden werden kann, und einem Auslass (411), der mit der Einlassöffnung (3) der Abgabevorrichtung (1) assoziiert werden kann; und wobei die erste Kompensationsleitung (50) ein erstes Ende (51), das mit dem zweiten Rohr (48) stromaufwärts der Patrone (4) verbunden werden kann, und ein zweites Ende (51) aufweist, das mit dem Ventil (6) der Abgabevorrichtung (1) verbunden ist.

2. Einheit nach Anspruch 1, **dadurch gekennzeichnet, dass** die Öffnung des Ventils (6) proportional zu der Druckdifferenz ist, die innerhalb der Kammer (2) und außerhalb der Kammer (2) an der ersten Einlassöffnung (3) herrscht, um den Druck innerhalb der Kammer (2) auf einem konstanten Wert zu halten.

3. Einheit nach Anspruch 1 oder 2, **dadurch gekennzeichnet, dass** die Öffnung des Ventils (6) einstellbar ist.

4. Einheit nach einem der vorhergehenden Ansprüche 1 bis 3, **dadurch gekennzeichnet, dass** das Ventil vom Ventilteller-Typ (6', 6", 600) ist, umfassend eine Aufnahme (16, 16", 160), vorzugsweise kreisförmig, mit einem ersten offenen Ende (17), das mit der Kammer (2) der Abgabevorrichtung (1) an der dritten Öffnung (7) verbunden ist, und einem zweiten offenen Ende (18), einen Schaft (13) mit einem im Wesentlichen flachen Kopf (14), der an einem ersten Ende (15) des Schafts (13) angeordnet ist und an der Öffnung des ersten Endes (17) der Aufnahme (16, 16", 160) angeordnet ist, eine Führungsstütze (20, 20", 200) für den Schaft (13), die innerhalb der Aufnahme (16, 16", 160) angeordnet ist, und eine Feder (21), die zwischen der Führungsstütze (20, 20", 200) und dem zweiten Ende (15') des Schafts (13) angeordnet ist.

5. Einheit nach Anspruch 4, **dadurch gekennzeichnet, dass** die Aufnahme (16", 160) des Ventils eine zumindest teilweise mit Gewinde versehene Innenwand (25) aufweist und die Führungsstütze (20", 200) des Schafts (13) eine zumindest teilweise mit Gegengewinde versehene Außenwand (26, 34) aufweist, um zur Veränderung der Federvorspannung innerhalb der Aufnahme verschraubt zu werden.

6. Einheit nach einem der vorhergehenden Ansprüche 1 bis 3, **dadurch gekennzeichnet, dass** das Kompensationsventil (6) vom Typ Entenschnabel-Rückschlagventil ist und innerhalb der Kammer (2) eingesetzt ist.

7. Abgabeeinheit nach einem der vorhergehenden Ansprüche 1 bis 6, **dadurch gekennzeichnet, dass** sie mindestens ein Bypass-Ventil (52) mit einem Einlass (53), einem ersten Auslass (54) und einem zweiten Auslass (55) umfasst, wobei der Einlass (53) mit dem zweiten Rohr (48) verbunden werden kann; wobei eine zweite Versorgungsleitung (56) ein erstes Ende (57), das mit dem ersten Auslass (54) des Bypass-Ventils (52) verbunden ist, und ein zweites Ende (58) aufweist, das mit dem Einlass (410) der Patrone (4) verbunden werden kann; und wobei eine dritte Bypass-Leitung (59) ein erstes Ende (60), das mit dem zweiten Auslass (55) des Bypass-Ventils (52) verbunden ist, und ein zweites Ende (61) aufweist, das mit dem ersten Rohr (42) verbunden werden kann.

## Revendications

1. Unité d'administration d'au moins un produit à inhaler comprenant un dispositif d'administration,
dans lequel ledit dispositif d'administration comprend au moins une chambre (2) ayant une première ouverture d'entrée (3) adaptée pour être connectée à une cartouche (4) contenant au moins un produit à inhaler, une deuxième ouverture de sortie (5) adaptée pour être connectée à au moins un élément d'aspiration de produit, la deuxième ouverture de sortie (5) étant disposée à une distance prédéterminée de la première ouverture d'entrée (3), dans lequel le dispositif comprend en outre au moins une soupape de compensation (6) disposée au niveau d'une troisième ouverture (7) pratiquée sur une paroi (8) de la chambre (2), la soupape de compensation (6) étant adaptée pour maintenir une configuration fermée lorsque la pression à l'intérieur de la chambre (2) est substantiellement égale à la pression dans un premier conduit de compensation (50) et une configuration ouverte lorsque la pression à l'intérieur de la chambre (2) est inférieure à une valeur prédéterminée,
dans laquelle la deuxième ouverture de sortie (5) est adaptée pour être connectée à un premier tuyau (42) ; au moins une cartouche (4) comprenant un élément contenant (36) pour un produit à inhaler, en particulier des particules de cristaux de sel micronisées de différentes tailles, ayant une entrée (410) adaptée pour être connectée à un deuxième tuyau (48) et une sortie (411) adaptée pour être associée à l'ouverture d'entrée (3) du dispositif d'administration (1) ; et le premier conduit de compensation (50) ayant une première extrémité (51) adaptée pour être connectée au deuxième tuyau (48) en amont de la cartouche (4) et une deuxième extrémité (51) connectée à la soupape (6) du dispositif d'administration (1).

2. Unité selon la revendication 1, **caractérisée en ce que** l'ouverture de la soupape (6) est proportionnelle à la différence de pression existant à l'intérieur de la chambre (2) et à l'extérieur de la chambre (2) au niveau de la première ouverture d'entrée (3), de manière à maintenir la pression à l'intérieur de la chambre (2) à une valeur constante.

3. Unité selon la revendication 1 ou 2, **caractérisée par le fait que** l'ouverture de la soupape (6) est réglable.

4. Unité selon l'une quelconque des revendications précédentes 1 à 3, **caractérisée en ce que** la soupape est du type à clapet (6', 6", 600), comprenant un contenant (16, 16", 160), de préférence circulaire, ayant une première extrémité ouverte (17) connectée à la chambre (2) du dispositif d'administration (1) au niveau de la troisième ouverture (7) et une deuxième extrémité ouverte (18), une tige (13) ayant une tête substantiellement plate (14) disposée à une première extrémité (15) de la tige (13) et disposée à l'ouverture de la première extrémité (17) du contenant (16, 16", 160), un support de guidage (20, 20", 200) pour la tige (13) disposé à l'intérieur de le contenant (16, 16", 160), et un ressort (21), interposé entre le support de guidage (20, 20", 200) et la deuxième extrémité (15') de la tige (13).

5. Unité selon la revendication 4, **caractérisée par le fait que** le contenant (16", 160) de la soupape a une paroi intérieure (25) au moins partiellement filetée et que le support de guidage (20", 200) de la tige (13) a une paroi extérieure (26, 34) au moins partiellement contre-filetée, de manière à être vissée à l'intérieur du contenant pour faire varier la précontrainte du ressort.

6. Unité selon l'une quelconque des revendications précédentes 1 à 3, **caractérisée par le fait que** la soupape de compensation (6) est du type soupape antiretour à bec de canard et est insérée à l'intérieur de la chambre (2).

7. Unité de d'administration selon l'une quelconque des revendications précédentes 1 à 6, **caractérisée en ce qu'**elle comprend au moins une soupape de dérivation (52) ayant une entrée (53), une première sortie (54) et une deuxième sortie (55), dans laquelle l'entrée (53) est adaptée pour être connectée au deuxième tuyau (48) ; un deuxième conduit d'alimentation (56) ayant une première extrémité (57) connectée à la première sortie (54) de la soupape de dérivation (52) et une deuxième extrémité (58) adaptée pour être connectée à l'entrée (410) de la cartouche (4) ; et un troisième conduit de dérivation (59) ayant une première extrémité (60) connectée à la deuxième sortie (55) de la soupape de dérivation (52) et une deuxième extrémité (61) adaptée pour être connectée au premier tuyau (42).
